# EUROPEAN PATENT SPECIFICATION

(11) **EP 2 236 625 B1**
(45) Date of publication and mention of the grant of the patent: **25.06.2014**
(21) Application number: 08855888.7
(22) Date of filing: 04.12.2008
(51) Int. Cl.: C12Q 1/68, A61B 5/00, G01N 33/50

(54) **USE OF TREFOIL FACTOR FAMILY 3 (TFF3) IN THE PROGNOSIS OF PATIENTS DIAGNOSED WITH COLORECTAL CANCER**
VERWENDUNG VON TFF3 (TREFOIL FACTOR FAMILY 3) BEI DER PROGNOSE VON PATIENTEN MIT KOLOREKTALKARZINOM-DIAGNOSE
UTILISATION DE TFF3 (TREFOIL FACTOR FAMILY 3) DANS LE PRONOSTIC DE SUJETS ATTEINTS DU CANCER COLORECTAL

(30) Priority: 04.12.2007 ES 200703230
(43) Date of publication of application: 06.10.2010
(73) Proprietor: Universidad Autónoma De Madrid, 28049 Madrid (ES); Fundación para la Investigación Biomédica del Hospital Universitario de la Paz, 28046 Madrid (ES); Consejo Superior De Investigaciones Científicases, 28006 Madrid (ES)
(72) Inventor: CASADO SÁENZ, Enrique, 28702 San Sebastián de los Reyes - Madrid (ES); CEJAS GUERRERO, Paloma, E-28046 Madrid (ES); SÁNCHEZ HERNÁNDEZ, José Javier, E-28049 Madrid (ES); GONZÁLEZ BARÓN, Manuel, E-28046 Madrid (ES); PERONA ABELLÓN, Rosario, E-28029 Madrid (ES)
(74) Representative: ABG Patentes, S.L.
(86) International application number: PCT/ES2008/000757
(87) International publication number: WO 2009/071721

(56) References cited:
- WO-A2-2005/013802
- WO-A2-2007/082099
- US-A1- 2007 172 857
- XIANYANG YIO ET AL: "Trefoil factor family-3 is associated with aggressive behavior of colon cancer cells", CLINICAL & EXPERIMENTAL METASTASIS ; OFFICIAL JOURNAL OF THEMETASTASIS RESEARCH SOCIETY, KLUWER ACADEMIC PUBLISHERS, DO, vol. 22, no. 2, 1 April 2005 (2005-04-01), pages 157-165, XP019235821, ISSN: 1573-7276, DOI: DOI:10.1007/S10585-005-6615-Z
- GARCIA ALFONSO P. ET AL.: 'Tratamiento paliativo dthe cancer colorrectal' CIR ESP, [Online] vol. 73, no. 1, 2003, pages 46 - 51 Retrieved from the Internet: <URL:http://www.cirugest.com/htm/revisiones /cirl5-14/15-14-20.pdf>
- TAUPIN D R ET AL: "Intestinal trefoil factor confers colonic epithelial resistance to apoptosis", PROCEEDINGS OF THE NATIONAL ACADEMY OF SCIENCES, NATIONAL ACADEMY OF SCIENCES, US, vol. 97, no. 2, 18 January 2000 (2000-01-18), pages 799-804, XP002258730, ISSN: 0027-8424, DOI: 10.1073/PNAS.97.2.799

## Description

### Field of the Invention

The present invention relates to *in vitro* methods for establishing a prognosis concerning the progress of a patient diagnosed with colorectal cancer after the administration of a therapy, monitoring the effect of said therapy on the patient or predicting the clinical response of said patient to the therapy with respect to basal expression levels.

### Background of the Invention

Large intestine cancer is the third most common malignant tumor in the Western world, following lung and breast cancer, representing 10% of all cancers and causing 10% of cancer-related deaths. Approximately a third of all large intestine cancers are formed in the rectum, and most of these cancers are diagnosed as locally advanced rectal cancers (LARCs).

In LARC, the development of the Total Mesorectal Excision (TME) and of preoperative strategies with chemotherapy and radiotherapy has considerably improved overall survival, local control and probably the proportion of procedures which preserve the anal sphincter. Furthermore, chemoradiotherapy (CRT) toxicity has thus been mitigated.

According to the data from old and recent randomized clinical trials, the combined preoperative treatment with CRT has extended overall 5 year survival from 45% with conventional surgery (Douglass, H.O., et al. 1986, N.Engl.J Med., 315: 1294-1295; Tveit, K.M., et al. 1997 Norwegian Adjuvant Rectal Cancer Project Group, Br.J Surg., 84: 1130-1135), to 66-76% with preoperative CRT, and local recurrences have decreased from 30-50% to 6-8%. However, a third of the patients will develop distant metastases, which are responsible for the elevated mortality (Sauer, R., et al. 2004 N.Engl.J.Med., 351: 1731-1740; Bosset, J.F., et al. 2006 N.Engl.J Med., 355: 1114-1123).

Furthermore, a significant number of patients suffer considerable adverse effects associated with CRT with no benefits whatsoever. In contrast, other patients can be under-treated. It is therefore very interesting to identify the potential targets involved in the resistance to CRT and to identify the patients who will relapse and could benefit from more intensive treatments.

The selection of the CRT treatment for each individual case of rectal adenocarcinoma depends on clinical and pathological variables which are unable to predict the therapeutic efficacy. Individual targeted strategies which correlate specific basal biomarkers with the response to the preoperative CRT treatment have shown markers of interest, including the mutations in p53 (Kandioler, D. et al., 2002. Ann.Surg., 235:493-498; Rebischung, C., et al. 2002, Int. J Cancer, 100: 131-135), expression of thymidylate synthase (Johnston, P.G., et al. 1994, J Clin Oncol, 12: 2640-2647; Edler, D. et al., 2000, Clin Cancer Res. 6:1378-1384), p21 (Reerink, O., et al. 2004, Anticancer Res., 24:1217-1221; Qiu, H., et al. 2000, Dis. Colon Rectum, 43: 451-459; Fu, C.G., et al. 1998, Dis. Colon Rectum, 41: 68-74; Rau, B., et al. 2003, J Clin Oncol, 21: 3391-3401), EGFR (Milas, L., et al. 2004, Int. J Radiat.Oncol Biol.Phys., 58: 966-971), COX 2 (Smith, F.M., et al. 2006 Int.J.Radiat.Oncol.Biol.Phys., 64: 466-472), rate of spontaneous apoptosis (Rodel, C., et al. 2002, Int. J Radiat.Oncol Biol.Phys., 52: 294-303; Abe, T., et al. 2001, Anticancer Res., 21: 2115-2120) or CEA (Park, Y.A., et al. 2006, J Surg.Oncol, 93: 145-150). However, none of them has clearly demonstrated their predictive usefulness in clinical practice.

Since exposure to CRT induces the clonal expansion of resistant cancer cells, the dynamic evaluation of markers which respond to CRT has sparked enormous interest in rectal cancer (Crane, C.H., et al. 2003, J Clin Oncol, 21: 3381-3382). Blind gene screening through this strategy can identify new prognostic and potentially therapeutic targets. Furthermore, comparisons between patients reduce the sources of variability, offering an attractive context for target screening.

Few clinical studies have approached the dynamic evaluation of individual markers before and after treatment. Rau *et al*. found in a series of 66 patients that the reduction in the immunohistochemical expression of p21, and the increase in the expression of Ki-67, was associated with a worse prognosis (Rau, B., et al. 2003, J Clin Oncol, 21: 3391-3401). Similarly, in 40 patients included in the mentioned German trial, the persistence of lymph node disease after treatment and the increase in the expression of thymidylate synthase therein, after laser microdissection of the tumor, was accompanied by a higher rate of recurrences (Liersch, T. et al. 2006, J Clin Oncol, 24: 4062-4068).

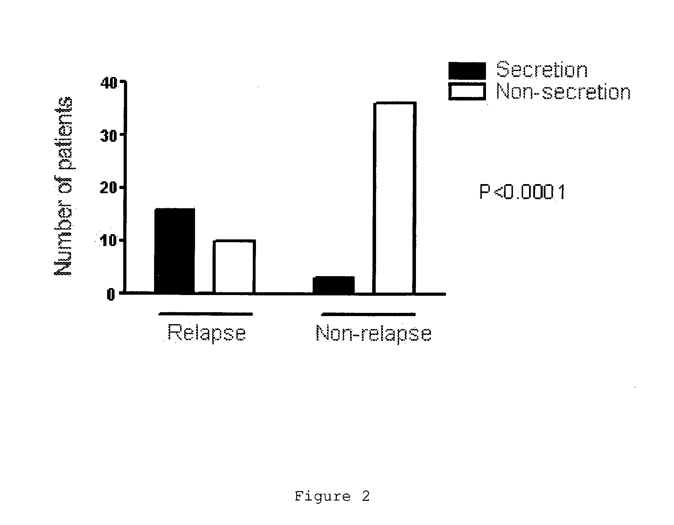

"Trefoil Factor Family 3" (TFF3), also called "Intestinal Trefoil Factor" (ITF), is a protein released by the goblet cells of the intestinal mucosa to the intestinal lumen (Figure 2). Its main function is to collaborate in repairing the intestinal epithelium. To that end, it acts on the epithelial reserve cells stimulating their division and migration for the re-epithelialization of the damaged surfaces where the epithelium has been lost. TFF3 activates a series of intracellular signaling pathways which induce proliferation, act on the cell adhesions to facilitate cell mobility, inhibit cell apoptosis and can even send angiogenesis activation signals to the endothelial cells of the mucosa and the lamina propria.

Knockout mice for TFF3 are phenotypically normal unless their intestine is damaged, in which case the mice die due to failure in healing the wound. The use of a recombinant TFF3 for the therapy related to irritable bowel syndrome and other bowel diseases (e.g., patent US 6,063,755, 6,316,218 and patent application US20010052483) has been proposed.

The physiological effects of TFF3 in intestinal cells can be considered beneficial for the growth of cancer cells, proliferation and metastasis. For example, it is believed that the resistance to apoptosis, induction of the migration and protection of the activation of the complement are mechanisms through which the cancer cells escape from the growth control, invade the adjacent normal tissues and escape from the vigilance of the immune system. The data collected from clinical practice suggest that the expression of TFF3 can be correlated with the poor prognosis of gastric cancer (Yamachika, et al., Clin. Cancer Res., 2002, 8, 1092).

Yio, X. et al., 2005 (Clin Exp Metastasis. Vol. 22(2): 157-165) describe the correlation between the endogenous and constitutive expression of TFF3 and an aggressive phenotype in colon cancer cells. More specifically, the inventors have observed that all the human colon metastases examined express TFF3 and that TFF3 confers a malignant behavior to colon cancer cells. Additionally, they mention conclusions of other inventors, among which the fact that in gastric cancer cells the inhibition of the expression of TFF3 causes a decrease in cell growth and an increased sensitivity to chemotherapy is pointed out.

In addition, Solmi, R. et al., 2004 (Int J Oncol. Col. 25(4):1049-56) describe a set of 11 genes, including TFF3, which are differentially expressed in colon cancer.

Patent application US2007/0172857, in the name of Sysmex Corporation, describes a set of proteins, among which TFF3 is pointed out, which are expressed in excess in cancer cells derived from colon cancer. After several PCR cycles, TFF3 is identified as an effective marker for detecting metastasis towards the lymph node of colon cancer.

International patent application WO2007/082099, in the name of Genomic Health, INC and NASBP Foundation, describes gene expression markers for the prognosis of colorectal cancer in different stages of development of the cancer. Among the markers described is TFF3, which is used in a method for predicting the clinical result in a patient diagnosed with cancer after the surgical resection of said cancer. Depending on the stage in which the cancer is located, i.e., Dukes B (stage II) or Dukes C (stage III), the expression level of TFF3 indicates a higher or lower probability of a positive clinical development for the patient.

Finally, Rau, U. et al., 2003 (J Clin Oncol. Vol. 21(18): 3391-401) describe the dynamic expression of two genes, P21WAF1/CIP1 and Ki-67 in patients with rectal carcinoma, before and after subjecting said patients to a preoperative treatment of radiochemotherapy. Thus, the authors found that the reduction of the immunohistochemical expression of p21 and the increase of the expression of ki-67, was associated with a worse prognosis of the patient.

Therefore, there is in the state of the art the need to provide a gene expression marker alternative to those already existing, which allows reliably establishing the prognosis for the response of a patient diagnosed with colorectal cancer to the therapy administered in the treatment of said cancer.

### Summary of the Invention

In one aspect, the present invention relates to an *in vitro* method for establishing a prognosis concerning the progress of a patient diagnosed with colorectal cancer after the administration of a therapy, comprising
(i) quantifying the expression levels of the *TFF3* gene, or
(ii) quantifying the levels of proteins encoded by said gene, or any amino-acid sequence that has at least 90% identity with said TFF3 protein,
in a biological sample from said patient before and after the therapy, wherein an increase of the expression of *TFF3* or of the expression level of the protein encoded by said gene, or any amino-acid sequence that has at least 90% identity with said TFF3 protein, after the administered therapy with respect to the expression of *TFF3* or the expression level of the protein encoded by said gene, or any amino-acid sequence that has at least 90% identity with said TFF3 protein, before the therapy, is indicative of a worse progress of the patient.

In another aspect, the invention relates to an *in vitro* method for monitoring the effect of the therapy administered to a patient diagnosed with colorectal cancer, comprising
(i) quantifying the expression levels of the *TFF3* gene, or
(ii) quantifying the levels of the protein encoded by said gene, or any amino-acid sequence that has at least 90% identity with said TFF3 protein,
in a biological sample from said patient before and after the administration of the therapy, wherein an increase of the expression of *TFF3* or of the expression level of the protein encoded by said gene, or any amino-acid sequence that has at least 90% identity with said TFF3 protein, after the administered therapy with respect to the expression of *TFF3* or the expression level of the protein encoded by said gene, or any amino-acid sequence that has at least 90% identity with said TFF3 protein, before the therapy, is indicative that the administered therapy is not effective.

In another aspect, the invention relates to an *in vitro* method for designing a customized therapy for a patient diagnosed with colorectal cancer, comprising
(i) quantifying the expression levels of the *TFF3* gene, or
(ii) quantifying the levels of the protein encoded by said gene, or any amino-acid sequence that has at least 90% identity with said TFF3 protein,
in a biological sample from said patient before and after the therapy, wherein an increase of the expression of *TFF3* or of the expression level of the protein encoded by said gene, or any amino-acid sequence that has at least 90% identity with said TFF3 protein, after the administered therapy with respect to the expression of *TFF3* or the expression level of the protein encoded by said gene, or any amino-acid sequence that has at least 90% identity with said TFF3 protein, before the therapy, is indicative that the patient needs an alternative therapy to the therapy originally administered.

In another aspect, the invention relates to an *in vitro* method for establishing a prognosis concerning the progress of a patient diagnosed with colorectal cancer, comprising determining the distribution pattern of the protein encoded by the *TFF3* gene, or any amino-acid sequence that has at least 90% identity with said TFF3 protein, in the tumor, wherein the detection of TTF3, or any amino-acid sequence that has at least 90% identity with said TFF3 protein, in the lumen of the tumor microglands and/or inside the lymph or blood vessels, is indicative of a worse progress of a patient.

Finally, in another aspect, a method for predicting the clinical response of a patient diagnosed with colorectal cancer to a therapy, comprising
(i) quantifying the expression levels of the *TFF3* gene or
(ii) quantifying the expression levels of the protein encoded by said gene, or any amino-acid sequence that has at least 90% identity with said TFF3 protein,
in a biological sample from said patient before the administration of the therapy, wherein elevated expression levels of *TFF3* with respect to the basal expression levels of *TFF3* are indicative of a poor clinical response of the patient to the therapy, is disclosed.

### Brief Description of the Drawings

Figure 1 depicts graphs showing the Overall survival (A) and the disease-free survival (B) depending on the alterations in the expression of TFF3 after the treatment with chemoradiotherapy.
Figure 2 is a graph describing the statistically significant relationship (P<0.0001) between the intense immunostaining of TFF3 in the secretion in the lumen of the microglands of the tumor and the risk of relapse of the patients.
Figure 3 are photographs showing the immunostaining of TFF3 in the secretion of the lumen of the tumor microgland. **A:** Case with low expression of TFF3 in the cytoplasms of the tumor cells. **B:** Case with high expression of TFF3 in the cytoplasms of the tumor cells. **C:** Immunostaining of TFF3 in the lumen of the vessel.

### Detailed Description of the Invention

The selection of the chemotherapy treatment for each individual case of colorectal cancer depends on clinical and pathological variables which are unable to predict the therapeutic efficacy of a treatment or to establish a prognosis concerning the progress of a patient suffering said type of cancer. The inventors have surprisingly found an increase of the expression levels of the *TFF3* gene or of the protein encoded by said gene, after the treatment with chemoradiotherapy (CRT) is associated with a high risk of relapse of the patient suffering from said cancer.

In one aspect, the disclosure relates to an *in vitro* method for establishing a prognosis concerning the progress of a patient diagnosed with colorectal cancer after the administration of a therapy, comprising
(i) quantifying the expression levels of the *TFF3* gene, or
(ii) quantifying the levels of the protein encoded by said gene, or any functionally equivalent variant of said TFF3 protein,
in a biological sample from said patient before and after the therapy, wherein an increase of the expression of *TFF3* or of the expression level of the protein encoded by said gene, or any functionally equivalent variant of said TFF3 protein, after the administered therapy with respect to the expression of *TFF3* or the expression level of the protein encoded by said gene, or any functionally equivalent variant of said TFF3 protein, before the therapy, is indicative of a worse progress of the patient.

In a particular embodiment of the *in vitro* method for establishing a prognosis concerning the progress of a patient diagnosed with colorectal cancer, the parameter indicating the progress of said patient is selected from the group of risk of relapse, disease-free survival and/or overall survival of the patient.

In the present invention, "risk of relapse" is understood as the probability of a patient to re-develop colorectal cancer after a disease-free period.

In the present invention, "disease-free survival" is understood as time period after treatment in which the cancer is not detected.

In the present invention, "overall survival of the patient" is understood as the percentage of patients who survive from the time of the diagnosis or treatment until the end of a defined time period.

In another aspect, the disclosure relates to an *in vitro* method for monitoring the effect of the therapy administered to a patient diagnosed with colorectal cancer, comprising
(i) quantifying the expression levels of the *TFF3* gene, or
(ii) quantifying the levels of the protein encoded by said gene, or any functionally equivalent variant of said TFF3 protein,
in a biological sample from said patient before and after the administration of said therapy, wherein an increase of the expression of *TFF3* or of the expression level of the protein encoded by said gene, or any functionally equivalent variant of said TFF3 protein, after the administered therapy with respect to the expression of *TFF3* or the expression level of the protein encoded by said gene, or any functionally equivalent variant of said TFF3 protein, before the therapy, is indicative that the therapy is not effective.

In another aspect, the disclosure relates to an *in vitro* method for designing a customized therapy for a patient diagnosed with colorectal cancer, comprising
(i) quantifying the expression levels of the *TFF3* gene, or
(ii) quantifying the levels of the protein encoded by said gene, or any functionally equivalent variant of said TFF3 protein,
in a biological sample from said patient, wherein an increase of the expression of *TFF3* or of the expression level of the protein encoded by said gene, or any functionally equivalent variant of said TFF3 protein, after the administered therapy with respect to the expression of *TFF3* or the expression level of the protein encoded by said gene, or any functionally equivalent variant of said TFF3 protein, before the therapy, is indicative that the patient needs an alternative therapy to the therapy originally administered.

As it is used herein, the term "protein" relates to a molecular chain of amino acids, bound by covalent or noncovalent bonds. The term furthermore includes all the forms of physiologically relevant post-translational chemical modifications, for example, glycosylation, phosphorylation or acetylation, etc., provided that the functionality of the protein is maintained.

As it is used herein, "functionally equivalent variant of the TFF3 protein" is understood as a protein the amino acid sequence of which (i) is substantially homologous to the amino acid sequence of the TFF3 protein and (ii) performs the same functions as the TFF3 protein. The functional similarity of one protein with another specific protein can be determined by means of interference assays with the expression of the gene encoding the specific protein which, upon reducing the expression, would reduce the activity of that protein and the subsequent recovery of the activity by means of expression of the sequence of the other protein. These experiments are performed using specific interference RNA sequences complementary for the sequence of the specific protein and expression vectors incorporating the specific sequence of the other protein regulated by an inducible or non-inducible promoter.

An amino acid sequence is substantially homologous to a determined amino acid sequence when it presents a degree of identify of at least 70%, advantageously of at least 75%, typically of at least 80%, preferably of at least 85%, more preferably of at least 90%, still more preferably of at least 95%, 97%, 98% or 99%, with respect to said determined amino acid sequence. The degree of identity between two amino acid sequences can be determined by conventional methods, for example, by means of standard sequence alignment algorithms known in the state of the art, such as, for example, BLAST [Altschul S.F. et al. Basic local alignment search tool. J Mol Biol. 1990 Oct 5; 215(3):403-10].

The person skilled in the art will understand that the mutations in the nucleotide sequence of *TFF3* which give rise to conservative substitutions of amino acids in non-critical positions for the functionality of the protein, are evolutionally neutral mutations which do not affect its overall structure or its functionality. Also included are those functionally equivalent variants of TFF3 presenting insertions, deletions or modifications of one or more amino acids with respect to TFF3, and furthermore conserving the same functions as TFF3.

Therefore, as it is used herein, the term "functionally equivalent variant" also includes any functionally equivalent fragment of a TFF3. The term "fragment" relates to a peptide comprising a part of a protein. In this case, a functionally equivalent fragment of TFF3 is a peptide or protein comprising a part of TFF3 and the same functions as TFF3.

In the present invention, "alternative therapy" is understood as a therapy that is different from the one originally administered, herein referred to as standard therapy or conventional therapy, to a patient. Said "alternative therapy" includes significant variations to the standard therapy, such as the substitution of some agents with others, the addition of alternative chemotherapy agents, change of doses or increase of the dose intensity of the drugs, addition of other anti-cancer agents (approved or in the experimental phase), alteration in the administration sequence of the anti-cancer agents or the type of local treatments, such as surgery or radiotherapy, etc. The alternative therapies herein defined are probably associated with more side effects for the patient (though not necessarily) and foreseeably greater effectiveness. Specific examples of anti-cancer agents included within the alternative therapy could be irinotecan, bevacizumab and cetuximab, agents which attack signaling pathways, antiangiogenic agents, etc.

In the present invention, "standard therapy" or "conventional therapy" is understood as any therapy that uses the drugs with clinical efficacy proven in randomized phase III studies, alone or in combinations similar to those used in the present invention, for example, in colorectal cancer the use of oxaliplatin, 5-fluorouracil or oral fluoropyrimidines, irinotecan, bevacizumab, or cetuximab, in regimens such as FOLFOX, FOLFIRI, XELOX, or XELIRI, with or without bevacizumab, or cetuximab, etc.

The expression levels of the *TFF3* gene can be quantified based on the RNA resulting from the transcription of said gene (mRNA) or, alternatively, based on the complementary DNA (cDNA) of said gene. Therefore, in a particular embodiment quantifying the expression levels of the *TFF3* gene comprises quantifying the messenger RNA of the *TFF3* gene, a fragment of said mRNA, complementary DNA of the *TFF3* gene, a fragment of said cDNA, or mixtures thereof. Additionally, the method of the invention can include performing an extraction step for the purpose of obtaining the total RNA, which can be done by means of conventional techniques (Chomczynski et al., Anal. Biochem., 1987, 162:156; Chomczynski P., Biotechniques, 1993, 15:532).

Virtually any conventional method can be used within the framework of the invention for detecting and quantifying the levels of mRNA encoded by the *TFF3* gene or of its corresponding cDNA. By way of non-limiting illustration, the levels of mRNA encoded by said gene can be quantified by means of the use of conventional methods, for example, methods comprising mRNA amplification and quantifying the product of said mRNA amplification, such as electrophoresis and staining, or alternatively by means of Southern blot and the use of suitable probes, Northern blot and the use of specific probes of the mRNA of the gene of interest (*TFF3*) or of its corresponding cDNA, mapping with S1 nuclease, RT-LCR, hybridization, microarrays, etc., preferably by means of real time quantitative PCR using a suitable marker. Similarly, the levels of the cDNA corresponding to said mRNA encoded by the *TFF3* gene can also be quantified by means of the use of conventional techniques; in this case, the method of the invention includes a step of synthesis of the corresponding cDNA by means of reverse transcription (RT) of the corresponding mRNA followed by amplification and quantification of the product of said cDNA amplification. Conventional methods for quantifying the expression levels can be found, for example, in Sambrook et al., 2001. "Molecular cloning: a Laboratory Manual", 3rd ed., Cold Spring Harbor Laboratory Press, N.Y., Vol. 1-3.

In a particular embodiment, the expression levels of the *TFF3* gene are quantified by means of a quantitative polymerase chain reaction (PCR).

In addition, besides quantifying the expression levels of the *TFF3* gene, the expression levels of the protein encoded by said gene, i.e., the TFF3 protein (TFF3), or any functionally equivalent variant of said TFF3 protein, can also be quantified. Thus, in a particular embodiment, quantifying the levels of the protein encoded by the *TFF3* gene comprises quantifying the TFF3 protein.

The expression level of the TFF3 protein can be quantified by means of any conventional method which allows detecting and quantifying said protein in a sample from a patient. By way of non-limiting illustration, the levels of said protein can be quantified, for example, by means of the use of antibodies with the ability to bind to TFF3 (or to fragments thereof containing an antigenic determinant) and subsequently quantifying the complexes formed. The antibodies which are used in these assays can be labeled or not. Illustrative examples of markers which can be used include radioactive isotopes, enzymes, fluorophores, chemiluminescent reagents, enzymatic substrates or cofactors, enzymatic inhibitors, particles, dyes, etc. There is a wide variety of known assays which can be used in the present invention which use non-labeled antibodies (primary antibody) and labeled antibodies (secondary antibody); these techniques include Western blot, ELISA (enzyme-linked immunosorbent assay), RIA (radioimmunoassay), competitive EIA (competitive enzymatic immunoassay), DAS-ELISA (double antibody sandwich ELISA), immunocytochemical and immunohistochemical techniques, techniques based on the use of protein biochips or microarrays including specific antibodies or assays based on colloidal precipitation in formats such as dipsticks. Other ways for detecting and quantifying said TFF3 protein include affinity chromatography techniques, ligand binding assays, etc. There are on the market commercial antibodies against TFF3 that can be used in the context of the present invention, such as, for example, the commercial monoclonal antibody against TFF3 Abnova H00007033-M01 and used in Example 2 attached to the present description.

In a particular embodiment, the levels of protein encoded by the *TFF3* gene are quantified by means of Western blot, ELISA, immunohistochemistry or ELISA.

In therapy for colorectal cancer, a variety of treatments can be used for attempting to treat or eliminate or containing the cancer. Depending on the patient's health, as well as on the size, site and stage of the cancer, (i) surgery, consisting of eliminating the part of the colon or rectum containing cancer as well as some of the surrounding healthy tissue, (ii) cytotoxic/cytostatic treatments, such as chemotherapy, using anti-cancer drugs to destroy the cancer cells by circulating the drugs throughout the body through the blood stream; radiotherapy, in which high-energy radiations are used to kill the cancer cells, and anti-tumor agents; and/or (iii) immunotherapy, in which the administered compound stimulates, enhances or repairs the natural function of the immunological system against cancer to recognize and eliminate the cancer cells from the body, can be used.

Therefore, in a particular embodiment, the administered therapy is a treatment cytotoxic and/ or cytostatic which, in yet another more particular embodiment, said treatment is chemotherapy, radiotherapy, antitumor agents or combinations thereof.

Suitable chemotherapy agents include but are not limited to alkylating agents such as, for example, cyclophosphamide, carmustine, daunorubicin, mechlorethamine, chlorambucil, nimustine, melphalan and the like; anthracyclines, such as, for example, daunorubicin, doxorubicin, epirubicin, idarubicin, mitoxantrone, valrubicin and the like; taxane compounds, such as, for example, paclitaxel, docetaxel and the like; topoisomerase inhibitors such as, for example, etoposide, teniposide, tuliposide , irinotecan and the like; nucleotide analogues such as, for example, azacitidine, azathioprine, capecitabine, cytarabine, doxifluridine, fluorouracil, gemcitabine, mercaptopurine, methotrexate, thioguanine, ftorafur and the like; platin-based agents such as, for example, carboplatin, cisplatin, oxaliplatin and the like; antineoplastic agents such as, for example, vincristine, leucovorin, lomustine, procarbazine and the like; hormonal modulators such as, for example, tamoxifen, finasteride, 5-α-reductase inhibitors and the like; vinca alkaloids such as, for example, vinblastine, vincristine, vindesine, vinorelbine and the like. The suitable chemotherapy agents are described in greater detail in the literature, such as in The Merck Index on CD-ROM, 13^{th} edition.

In a particular embodiment, chemotherapy comprises a compound selected from an alkylating agent preventing replication, a fluoropyrimidine, a thymidylate synthase inhibitor, a topoisomerase inhibitor and combinations thereof. Preferably, the alkylating agent preventing replication is oxaliplatin, the fluoropyrimidine is 5-fluorouracil, UFT, Utefos or Capecitabine, the thymidylate synthase inhibitor is raltitrexed, and the topoisomerase I inhibitor is irinotecan.

In the present invention, "antitumor agent" is understood as that physical or biological chemical agent or compound with antiproliferative, antioncogenic and/or carcinostatic properties that can be used for inhibiting the growth, proliferation and/or development of tumors. Examples of antitumor agents that can be used in the present invention are (i) antimetabolites, such as antifolates and purine analogues; (ii) natural products, such as antitumor antibiotics and mitotic inhibitors; (iii) hormones and antagonist thereof, such as androgens and corticosteroids; and (iv) biological agents, such as viral vectors. A list of compounds that can be used as antitumor agents is described in patent application WO2005/112973

In a particular embodiment, the antitumor agent comprises antiangiogenic agents and signaling pathway inhibitors which, in another more particular embodiment, the antiangiogenic agent is bevacizumab and the signaling pathway inhibitor is cetuximab, panitumumab, or erlotinib.

Putting the method into practice comprises obtaining a biological sample from the patient to be studied. Illustrative, non-limiting examples of said samples include different types of biological fluids, such as blood, serum, plasma, cerebrospinal fluid, feces, urine and saliva, as well as tissue samples. The samples of biological fluids can be obtained by any conventional method as can the tissue samples; by way of illustration, said tissue samples can be biopsy samples obtained by surgical resection.

Therefore, in a particular embodiment, the biological sample is a tissue sample or a biological fluid which, in yet another more particular embodiment is a tumor tissue sample.

Additionally, in another particular embodiment, the quantification of the expression levels of the protein encoded by the *TFF3* gene, or any functionally variant of said protein, can be measured in the cytoplasm of the tumor cells from the tumor tissue sample.

The method of the invention can be applied to any stage of colorectal cancer, from stage I to IV. Nevertheless, in a particular embodiment, the colorectal cancer is large intestine adenocarcinoma in stage II or III or IV.

Additionally, by means of the biopsy of the tumor, the inventors have found that the spatial distribution of the TFF3 protein in the tumor tissue shows a pattern that is correlated with the prognosis of a patient diagnosed with colorectal cancer. Thus, the presence of TFF3 in the lumen of the tumor microglands and/or inside the lymph or blood vessels is indicative of a worse progress of a patient.

Therefore, in another aspect the disclosure relates to an *in vitro* method for establishing a prognosis concerning the progress of a patient diagnosed with colorectal cancer, comprising determining the distribution pattern of the protein encoded by the *TFF3* gene in the tumor, or any functionally equivalent variant of said TFF3 protein, wherein the detection of TFF3, or any functionally equivalent variant of said TFF3 protein, in the lumen of the tumor microglands and/or inside the lymph or blood vessels, is indicative of a worse progress of a patient.

As has been previously indicated, the parameter indicating the progress of a patient diagnosed with colorectal cancer is selected from the group of risk of relapse, disease-free survival and/or overall survival of the patient, terms that have been previously defined.

In another particular embodiment, chemoradiotherapy is administered to the patient diagnosed with colorectal cancer before the extraction of the tumor.

Immunohistochemistry techniques allow the identification, on tissue or cytological samples, of antigenic determinants characteristic of different lines of cell differentiation and functionalism. The direct application of polyclonal or monoclonal antibodies on tissue sections allows the microanatomic location of their expression and their correlation with morphological parameters.

Thus, in another particular embodiment, the detection of protein encoded by the *TFF3* gene is performed by means of immunohistochemistry.

In another particular embodiment, the colorectal cancer is large intestine adenocarcinoma in stages II or III or IV.

In another aspect, the disclosure relates to a method for predicting the clinical response of a patient diagnosed with colorectal cancer to a therapy comprising
(i) quantifying the expression levels of the *TFF3* gene or
(ii) quantifying the expression levels of the protein encoded by said gene, or any functionally equivalent variant of said TFF3 protein,
in a biological sample from said patient before the administration of the therapy, wherein elevated expression levels of *TFF3* with respect to the basal expression levels of *TFF3* are indicative of a poor clinical response of the patient to the therapy.

In the present invention "basal expression levels" is understood as the expression level of *TFF3* in intestinal epithelial cells proliferating normally, i.e., without giving rise to the development of a tumor.

Additionally, in the present invention "basal expression levels" can also be understood as the expression level resulting from calculating the mean of the expressions of *TFF3* in a set of colorectal cancer tumor samples.

As the person skilled in the art will understand, the different treatments against cancer, the different techniques of the state of the art for quantifying the expression levels of a gene or of its corresponding protein mentioned in the present description can be applied to the present method and form, therefore, particular embodiments thereof.

### EXAMPLE 1

### Analysis of the dynamic change in the transcriptome after chemoradiotherapy

### I. MATERIAL AND METHODS

### Patients

The initial screening of changes in the transcriptome following CRT was performed on 25 patients who were subjected to a trial with preoperative CRT, after which fresh tumor samples were taken. The chemotherapy consisted of four preoperative and postoperative cycles of oxaliplatin and raltitrexed. Only 6 of the 25 patients were selected for the dynamic analysis by means of serial analysis of gene expression (SAGE), from whom only 3 suitable SAGE libraries were obtained.

Patient A presented an adverse progress, paradigmatic of resistance to CRT. The other two patients, B and C, presented tumors representative of the tumor regression grades (TRG) II-III, the most common. Patient A started with a rectal adenocarcinoma in a favorable clinical stage (uT3N0), adjacent to the anal margin, which required abdominoperineal resection. Tumor substaging was not obtained, the TRG was 2, and the distant and local recurrences were recorded at 16 and 48 months respectively.

Patients B and C presented disease with a clinical stage uT3N0 at the time of the diagnosis, the first disease-free patient being found at the end of five years, and the second one with pulmonary metastases 54 months after starting the treatment.

For the study of multiplex quantitative PCR (qRT-PCR), 129 additional patients with stages II-III of rectal adenocarcinoma under 10 centimeters from the anal margin, who received two or more chemotherapy cycles and 50.4 Gy of radiotherapy before surgery, were recruited prospectively and the clinical-pathological variables were recorded (see Table 1 below).

**Table 1: Patients and tumor characteristics**

| **Table 1.** | | | | | | | | |
|---|---|---|---|---|---|---|---|---|
| **Patients and tumor characteristics** | **Total** | | **TRG 0/1** | | **TRG 2/3** | | **TRG 4** | |
| | | | **NTR** | | **TR** | | | |
| | **n** | **%** | **n** | **%** | **n** | **%** | **n** | **%** |
| **Total number of patients** | 94 | 100 | 17 | 18.1 | 64 | 68.1 | 13 | 13.8 |
| **Mean age (range)** | 63(23 -84) | | 65(37-75) | | 64 (23-84) | | 63(45-73) | |
| **Sex** | | | | | | | | |
| Men | 54 | 57.4 | 10 | 58.8 | 37 | 57.8 | 7 | 53.8 |
| Women | 40 | 42.6 | 7 | 41.2 | 27 | 42.2 | 6 | 46.2 |
| **Anal distance** | | | | | | | | |
| <6 cm | 57 | 60.6 | 12 | 70.6 | 41 | 64.1 | 4 | 30.8 |
| >6 cm | 37 | 39.4 | 5 | 29.4 | 23 | 35.9 | 9 | 69.2 |
| **Post-CRT pathological tumor classification (yp T)** | | | | | | | | |
| ypT0 | 15 | 15.9 | 0 | 0 | 3 | 4.7 | 12 | 92.3 |
| ypT1 | 4 | 4.2 | 1 | 5.9 | 3 | 4.7 | - | - |
| ypT2 | 21 | 22.4 | 4 | 23.5 | 17 | 26.6 | - | - |
| ypT3 | 52 | 55.4 | 11 | 64.7 | 40 | 62.5 | 1 | 7.7 |
| ypT4 | 2 | 2.1 | 1 | 5.9 | 1 | 1.5 | - | - |
| **Post-CRT pathological node classification (yp N)** | | | | | | | | |
| ypN0 | 76 | 80.9 | 11 | 64,7 | 52 | 81.3 | 13 | 100 |
| ypN1 | 12 | 12,9 | 3 | 17.6 | 9 | 14.2 | 0 | 0 |
| ypN2 | 6 | 6.5 | 3 | 17.7 | 3 | 4.7 | 0 | 0 |
| **Preoperative CRT** | | | | | | | | |
| Oxaliplatin-Raltitrexed | 49 | 52.1 | 5 | 29.4 | 37 | 57.8 | 7 | 53.8 |
| 5-FU in infusion/UFT | 45 | 47.9 | 12 | 70.6 | 27 | 42.2 | 6 | 46.2 |
| **Recurrence patterns** | | | | | | | | |
| Local | 6 | 21.4 | 2 | 22.2 | 4 | 20 | 0 | 0 |
| Distant | 20 | 71.4 | 6 | 66.6 | 15 | 75 | 1 | 100 |
| Both | 2 | 7.2 | 1 | 11.2 | 1 | 5 | 0 | 0 |
| **Death** | | | | | | | | |
| Yes | 14 | 14.9 | 4 | 23.5 | 10 | 15.6 | 0 | 0 |
| No | 80 | 85.1 | 13 | 76.5 | 54 | 84.4 | 13 | 100 |

| | | | | | | | | |
|---|---|---|---|---|---|---|---|---|
| CRT: Chemoradiotherapy; TR: Tumor response; NTR: No tumor response; TRG: Tumor regression grade | | | | | | | | |

94 patients were included in the statistical analysis, 34 of whom were discarded as a result of incomplete gene expression data or inappropriate pathological samples.

The dynamic evaluation of the changes in the gene expression was analyzed in tumors from 74 patients, 21 of which were rejected because they were cases of complete pathological response or of lacking sufficient tumor material for the analysis.

In all the cases, informed consents were obtained, and the study was previously approved by the corresponding hospital ethics committee.

### Follow up

Follow-up was performed on the patients at three-month intervals for two years, after that every six months for three years and after that, yearly. The evaluations consisted of a physical examination, hemogram and biochemical study. Colonoscopy, echography and computed tomography were performed according to standard guidelines (http://www.asco.org/asco/downloads/Colon_Cancer_Tool_11-1-05.xls.).

Whenever possible, a histopathological confirmation of recurrence was recommended.

### Preparation of the samples

For the SAGE analysis, fresh tumor samples were taken from the biopsies prior to and after CRT in the six patients selected. The tumor biopsies were frozen for 15 minutes with liquid nitrogen at -70°C. To isolate the RNA, the tumor samples stained with hematoxylin and eosin were examined by a pathologist; the frozen samples from the endoscopic biopsies were subjected to five micron serial cuts in their entirety; 20 sections with the same thickness were made in the frozen surgical specimens. It was required that all the samples contained at least 90% of tumor samples after the microdissection.

Out of the previously mentioned 129 recruited patients, 94 with complete clinical-pathological information and sufficient tumor sample for the analysis with low density arrays by means of multiplex quantitative PCR were selected. The paraffin-embedded samples were stained with hematoxylin-eosin and analyzed by a pathologist. Tumor cell enrichment greater than 75% was required, and where necessary, a subsequent macrodissection with a safety knife and a new hematoxylin-eosin staining in the postoperative biopsies were performed. The RNA was extracted from 5-10 five micron sections of the paraffined samples.

### Libraries and SAGE analysis

Six SAGE libraries were generated with the tumor samples from the endoscopic biopsies upon diagnosis and from the posttreatment surgical specimens from the three patients selected. The sections were directly homogenized and the resulting lysate was used directly for the modified SAGE methodology (microSAGE), following the previously described protocol (Datson, 1300-7) with minor modifications. A step of heating at 65°C for 10 minutes followed by two minutes in ice to allow a better separation of the concatemers was introduced. The products larger than 400 bp and smaller than 1,000 bp were cleaved, extracted and cloned in the Sphl site of the pZerO-1 vector marketed by the company (Invitrogen). The sequence files were analyzed using the SAGE 2000 program (kindly supplied by Kenneth W. Kinzler, Johns Hopkins University), and the resources of the webpage of the National Center for Biotechnology SAGE (http://www.ncbi.nlm.nih.gov/SAGE). Approximately 60,000 tags for the whole project, about 10,000 tags per SAGE library, were sequenced. To compare these SAGE libraries, each tag number was normalized with the SAGE 2000 program. The analysis of statistical significance was performed using the Monte Carlo analysis, which allows performing a large number of comparisons based on the results of the SAGE. The mapping between tags and genes was performed according to the SAGE Genie database, with reference to SAGEmap.

The genes which were regulated significantly after CRTs were selected for the subsequent exploratory phase in a wide patient series by means of multiplex quantitative PCR. This selection was carried out based on the statistically significant changes of the expression according to the Monte Carlo analysis (p<0.05), the similar tendency of the regulation before and following treatment (sub- or overregulation) in three patients, and finally the biological plausibility according to the earlier available literature.

### Multiplex quantitative PCR

The entire RNA content was measured and verified spectrophotometrically and electrophotometrically. The reverse transcription was performed based on 200 ng of total RNA using the High-Capacity cDNA Archive Kit (Applied Biosystems, Foster City, CA). The multiplex quantitative PCR reactions were performed in 384-well plates by means of the ABI PRISM 7900 HT Sequence Detection System (Applied Biosystems) in 50 µl samples with TaqMan Universal PCR Master Mix (Applied Biosystems) and 50 µl of cDNA corresponding to 50 ng of total RNA per channel of microfluidic card. The expression of each gene was measured in triplicate, and normalized in relation to three reference genes (GAPDH, B2M, and PMSB4). The explored genes included 25 obtained from the screening by means of SAGE (Table 2), and the rest from the literature (Table 3).

**Table 2. Blind screening of changes in the transcriptome after CRT by means of SAGE**

| **Gene** | **Patient A Pre/post (p)** | **Patient B Pre/Post (p)** | **Patient C Pre/post (p)** | **Unigene ID** | **Description** |
|---|---|---|---|---|---|
| MYO5B | 57/23 | 54/43 | 92/23 | 550481 | _{F}Myosin VB |
| | =0 | 0.11 | =0 | | |
| XIST | 4/11 | 3/12 | 4/11 | 529901 | X (inactive)-specific traffic |
| | 0.0665 | 0.02 | 0.056 | | |
| S100A10 | 8/13 | 13/4 | 2/8 | 143873 | S100 calcium binding protein A10 |
| | 0.21739 | 0.01944 | P=0.0457 | | |
| K-ALPHA-1 | 4/9 | 3/10 | 3/11 | 524390 | Tubulin, alpha, ubiquitous |
| | 0.1323 | 0.0529 | 0.02292 | | |
| HLA-C | 12/15 | 7/17 | 6/22 | 534125 | Major histocompatibility complex, class I, C |
| | 0.4310 | 0.0397 | 0.00118 | | |
| COL1A1 | 5/37 | 10/34 | 18/39 | 172928 | Collagen, type I, alpha |
| | =0 | 0.00030 | 0.00245 | | |
| TFF3 | 26/10 | 15/15 | 21/5 | 82961 | Trefoil factor 3 (intestinal) |
| | 0.00456 | 0.7 | 0.00173 | | |
| FXYD3 | 86/36 | 52/16 | 4/22 | 301350 | FXYD domain contains iron transport regulator 3 |
| | =0 | =0 | 0.00012 | | |
| CKB | 40/14 | 25/11 | 10/14 | 173724 | Creatinine kinase, brain |
| | 0.00025 | 0.0101 | 0.23 | | |
| CCNB1IP1 | 123/99 | 104/79 | 81/100 | 107003 | Cyclin B1 interacting protein 1 |
| | 0.04737 | 0.0266 | 0.05148 | | |
| HIG2 | 13/14 | 17/7 | 9/7 | 521171 | Hypoxia-inducing protein 2 |
| | 0.5348 | 0.02536 | 0.44 | | |
| PH-4 | 0/5 | 0/5 | 0/3 | 271224 | Factor prolyl-4-hydroxylase Hypoxia-inducing protein 2 |
| | 0.032765 | 0.03384 | 0.1165 | | |
| CHC1 | 7/1 | 6/3 | 2/1 | 469723 | Chromosome condensation 1 |
| | 0.032457 | 0.22 | 0.5 | | |
| BAT1 | 7/1 | 4/1 | 3/2 | 254042 | HLA-B associated transcript 1 |
| | 0.032457 | 0.1675 | 0.5122 | | |
| SELENBP1 | 15/5 | 14/5 | 20/6 | 334841 | Selenium binding protein 1 |
| | 0.018875 | 0.0304 | 0.0068 | | |
| ANGPTL4 | 9/4 | 5/6 | 12/4 | 9613 | Angiopoietin-like 4 |
| | 0.11 | 0.5 | 0.052 | | |
| SMAD2 | 258/185 | 266/178 | 297/184 | 465061 | SMAD, mothers against DPP homolog 2 (Drosophila) |
| | 0.00013 | =0 | 0.00001 | | |
| ZYX | 8/23 | 9/13 | 6/16 | 490415 | Zyxin |
| | 0.006 | 0.3 | 0.022 | | |
| GPX2 | 71/23 | 46/17 | 13/16 | 2704 | Glutathione peroxidase 2 (gastrointestinal) |
| | =0 | =0 | 0.3381 | | |
| MMP14 | 4/10 | 2/12 | 3/15 | 2399 | Matrix metalloproteinase 14 |
| | 0.0815 | 0.006 | 0.00311 | | (inserted-membrane) |
| ITGB4 | 0/6 | 1/5 | ¾ | 370255 | Integrin, beta 4 |
| | 0.017 | 0.1035 | 0.48 | | |
| WARS | 9/2 | 9/1 | 5/3 | 497599 | Tryptophanyl-tRNA synthetase |
| | 0.0354 | 0.0091 | 0.36992 | | |
| PCYT2 | 41/20 | 23/8 | 3/11 | 514635 | Phosphate cytidylyltransferase 2, ethanolamine |
| | 0.0039 | 0.0045 | 0.02292 | | |
| GSTP1 | 13/10 | 16/7 | 8/12 | 523836 | Glutathione S-transferase pi |
| | 0.324 | 0.0458 | 0.20702 | | |
| EEF2 | 52/34 | 45/32 | 40/37 | 515070 | Eukaryotic translation elongation factors |
| | 0.0257 | 0.0754 | 0.48 | | |

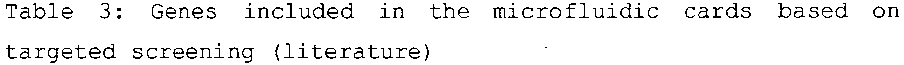

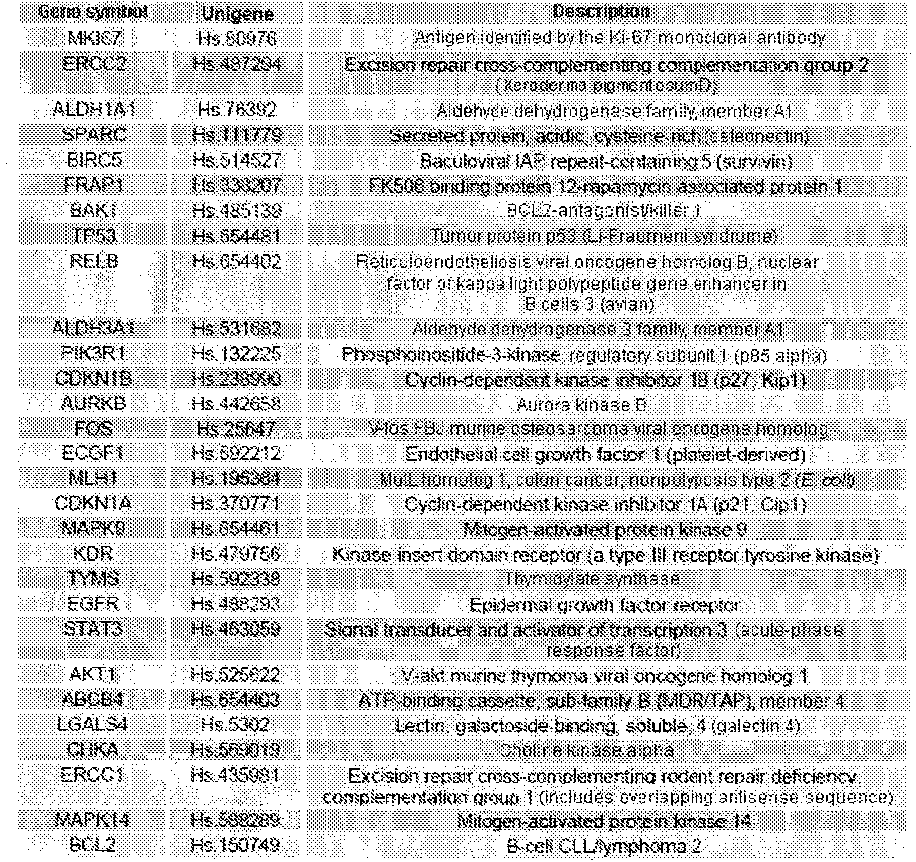

### Statistical analysis

*Descriptive statistics.* The absolute frequencies of onset of each modality of the variable and the relative frequencies expressed in percentages were calculated in the case of qualitative variables. The measurements of median or mean central tendency were calculated for the quantitative variables. The standard deviation and the range of the variable were used as measures of dispersion.

*Inferential statistics.* The chi square test with Yates' correction was applied for qualitative variables and Fisher's exact test was applied in the case of 2x2 Tables. Before applying the hypothesis test for quantitative variables, the Kolmogorov-Smirnov (KS) test was applied to study the type of distribution the variable followed (normal, non-normal distribution). For those variables in which the KS test indicated that it followed a normal distribution, parametric tests (Student's t), and in the other case non-parametric tests (Wilcoxon, Mann-Whitney U), were applied. As one of the objectives of the study is the assessment of the change in the expressions of genes before and after treatment with chemotherapy, part of the tests applied corresponded with tests for paired or dependent samples. The Kaplan-Meier method was used to estimate the overall survival.

### Normalization of Cₜ.

Data from the PCR-QT were normalized as a step prior to the analysis thereof. Two models were used for that purpose, which models were subsequently evaluated; a) normalizing with three genes and b) normalizing with 12 genes. The first method was applied due to the variability obtained with the type b) normalization. In all the cases it was normalized by the mean of each gene calculated in the entire patient series.

### Correlation of the dynamic changes in the data from qRT-PCR with prognosis.

The Wilcoxon test was used to study the changes in the expression of the genes analyzed after chemotherapy. Empirical p values for each gene were obtained through 5,000 permutation tests. The genes with statistically significant differences in the expression (p<0.01) were selected for discriminant and Cox's proportional hazards regression analysis. Cox's multivariate proportional hazards regression analysis (adjusted for sex, stage and number of positive ganglions) was performed for each gene in the training set (60% of the sample, the remaining 40% was the testing set). The assumption of proportional hazards for the covariables was investigated by examining the Schoenfeld residuals.

### II. RESULTS

### Clinical and pathological data

The histopathological and gene expression data were obtained in 94 patients for the multiplex quantitative PCR analysis. They are illustrated in Table 1.

### Screening of changes in the transcriptome after CRT by means of SAGE

Six libraries of genes in patients A, B and C were generated. The comparison of evolutionary changes in the transcriptome before and after treatment, through the Monte Carlo analysis, showed those which were significantly regulated. According to this analysis, a collection of genes with changes in their expression common in the three patients, in the same positive or negative regulation sense, the biological plausibility furthermore being considered, was selected. 17 genes were unidirectionally regulated showing p <0.05 in at least two of the patients, and statistical significance was achieved in one of the patients in seven genes. 25 genes were thus selected for their subsequent evaluation in a wide patient series (Table 2).

### Screening of transcriptional changes after CRT by means of Multiplex quantitative PCR

The analysis of alterations in the expression of the genes included in the microfluidic cards, before and after the treatment, showed a series of genes which were significantly regulated after the treatment (Table 4).

**Table 4. Genes which are significantly regulated after the chemoradiotherapeutic treatment**

| Gene | Difference | p |
|---|---|---|
| 4342379-18S (FAM,NFQ) | 1.81 | 0.0001 |
| ACTB-Hs99999903_m1 (FAM,NFQ) | 1.71 | 0.0001 |
| ABCB4-Hs00240956_m1 (FAM,NFQ) | 0.4 | 0.07 |
| AKT1-Hs00178289_m1 (FAM,NFQ) | 0.74 | 0.02 |
| ALDH1A1-Hs00167445_m1 (FAM,NFQ) | 2.07 | 0.0001 |
| ALDH3A1-Hs00167469_m1 (FAM,NFQ) | 0.58 | 0.04 |
| AURKB-Hs00177782_m1 | -0.59 | 0.11 |
| (FAM,NFQ) | | |
| BAK1-Hs00832876_g1 (FAM,NFQ) | 0.63 | 0.13 |
| BCL2-Hs00153350_m1 (FAM,NFQ) | 1.2 | 0.0001 |
| BIRC5-Hs00153353_m1 (FAM,NFQ) | -0.75 | 0.06 |
| CDKN1A-Hs00355782_m1 (FAM,NFQ) | 2.02 | 0.0001 |
| CDKN1B-Hs00153277_m1 (FAM,NFQ) | 0.89 | 0.01 |
| CHKA-Hs00608045_m1 (FAM,NFQ) | 0.15 | 0.62 |
| DPYD-Hs00559278_m1 (FAM,NFQ) | 1.8 | 0.0001 |
| ECGF1-Hs00157317_m1 (FAM,NFQ) | 0.96 | 0.008 |
| EGFR-Hs00193306_m1 (FAM,NFQ) | 0.79 | 0.03 |
| ERCC1-Hs00157415_m1 (FAM,NFQ) | 1.25 | 0.0001 |
| ERCC2-Hs00361161_m1 (FAM,NFQ) | 0.48 | 0.11 |
| FOS-Hs00170630_m1 (FAM,NFQ) | 3.58 | 0.0001 |
| FRAP1-Hs00234508_m1 (FAM,NFQ) | 0.24 | 0.47 |
| KDR-Hs00176676_m1 (FAM,NFQ) | 0.15 | 0.67 |
| LGALS4-Hs00196223_m1 (FAM,NFQ) | -1.08 | 0.03 |
| MAPK14-Hs00176247_m1 (FAM,NFQ) | 0.34 | 0.26 |
| MAPK9-Hs00177102_m1 | 0.43 | 0.21 |
| (FAM,NFQ) | | |
| MKI67-Hs00606991_m1 (FAM,NFQ) | -0.61 | 0.1 |
| MLH1-Hs00179866_m1 (FAM,NFQ) | 0.85 | 0.01 |
| PIK3R1-Hs00236128_m1 (FAM,NFQ) | 1.06 | 0.003 |
| RELB-Hs00232399_m1 (FAM,NFQ) | 1.11 | 0.001 |
| SPARC-Hs00234160_m1 (FAM,NFQ) | 2.38 | 0.0001 |
| STAT3-Hs00374280_m1 (FAM,NFQ) | 1.07 | 0.001 |
| TP53-Hs00153340_m1 (FAM,NFQ) | 0.35 | 0.43 |
| TYMS-Hs00426591_m1 (FAM,NFQ) | 0.22 | 0.52 |
| 4342379-18S (FAM,NFQ) | 2.56 | 0.0001 |
| ACTB-Hs99999903_m1 (FAM,NFQ) | 0.74 | 0.13 |
| ANGPTL4-Hs00211522_m1 (FAM,NFQ) | 0.43 | 0.007 |
| BAT1-Hs00366447_m1 (FAM,NFQ) | 0.99 | 0.02 |
| CCNB1IP1-Hs00603841_m1 (FAM,NFQ) | 1.47 | 0.002 |
| CHC1-Hs00154399_m1 (FAM,NFQ) | 1.58 | 0.0001 |
| CKB-Hs00176484_m1 (FAM,NFQ) | 2.24 | 0.0001 |
| COL1A1-Hs00164004_m1 (FAM,NFQ) | 1.5 | 0.001 |
| EEF2-Hs00157330_m1 | 0.98 | 0.009 |
| (FAM,NFQ) | | |
| FXYD3-Hs00254209_m1 (FAM,NFQ) | 0.62 | 0.11 |
| GPX2-Hs00702173_s1 (FAM,NFQ) | 0.17 | 0.71 |
| GSTP1-Hs00168310_m1 (FAM,NFQ) | 1.89 | 0.0001 |
| HIG2-Hs00203383_m1 (FAM,NFQ) | 3.31 | 0.0001 |
| HLA-C-Hs00740298_g1 (FAM,NFQ) | 1.27 | 0.008 |
| ITGB4-Hs00173995_m1 (FAM,NFQ) | -1.2 | 0.007 |
| K-ALPHA-1-Hs00744842_sH (FAM,NFQ) | 1.12 | 0.04 |
| MMP14-Hs00237119_m1 (FAM,NFQ) | -1.46 | 0.15 |
| MYO5B-Hs00393037_m1 (FAM,NFQ) | 0.62 | 0.12 |
| PCYT2-Hs00161098_m1 (FAM,NFQ) | 0.62 | 0.05 |
| PH-4-Hs00214665_m1 (FAM,NFQ) | -5.93 | 0.0001 |
| PMM1-Hs00160195_m1 (FAM,NFQ) | -0.05 | 0.9 |
| PSMB4-Hs00160598_m1 (FAM,NFQ) | 0.32 | 0.45 |
| PSMB6-Hs00382586_m1 (FAM,NFQ) | 1.72 | 0.0001 |
| S100A10-Hs00237010_m1 (FAM,NFQ) | 0 | - |
| SELENBP1-Hs00187625_m1 | 0.28 | 0.52 |
| (FAM,NFQ) | | |
| SMAD2-Hs00183425_m1 (FAM,NFQ) | 0.67 | 0.05 |
| TTF3-Hs00173625_m1 (FAM,NFQ) | 1.08 | 0.009 |
| WARS-Hs00188259_m1 (FAM,NFQ) | 1.08 | 0.009 |
| XIST-Hs00300535_s1 (FAM,NFQ) | 1.38 | 0.001 |
| ZYX-Hs00170299_m1 (FAM,NFQ) | 1 | 0.05 |

### Correlation of dynamic changes in the expression with the prognosis

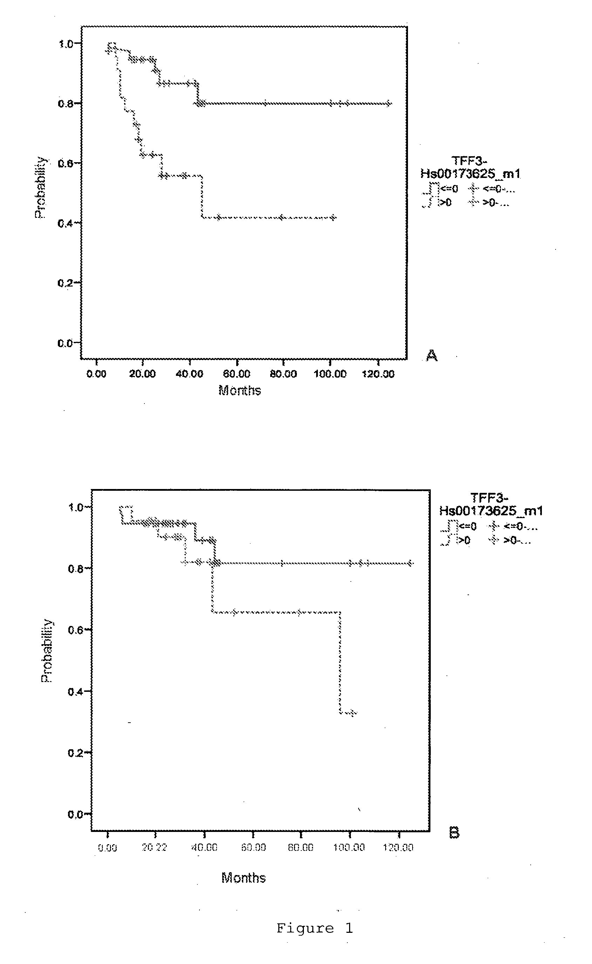

The analysis of the dynamic changes in the expression of the genes included in the microfluidic cards, in relation to the relapses of rectal cancer, showed that the positive regulation of TFF3 after the treatment was associated with a statistically significant pronounced tendency to relapse (p=0.005), as shown in Figure 1. Said Figure shows the disease-free survival (Figure 1A) and overall survival (Figure 1B). There was also a clear difference in the survival curves, although it does not reach statistical significance, in the probable context of a follow-up of less than 5 years, as is the case, given the mean survival of the relapses, of 20-30 months, in the disseminated disease.

### II. DISCUSSION

It is demonstrated in the present invention that a strategy of blind screening of the changes in the tumor transcriptome which develop after chemoradiotherapy, followed by a targeted exploration of these changes and other targets suggested in the literature before and after the treatment, is effective for finding a dynamic marker which identifies patients with high risk of relapse. It is therefore an interesting therapeutic target, particularly given the high biological plausibility derived from the biology of this candidate target, TFF3.

The great tendency to relapse of the patients who upregulate TFF3 after the treatment identifies patients with poor prognosis who could either benefit from alternative treatments to the standard treatment, in the preoperative context of the located disease or the disseminated disease. The data collected herein clearly show that TFF3 can be used for prognosis purposes and furthermore disclose the clinical interest that its approach may have, very compatible with the higher aggressiveness suggested in the behavior of colon cancer cell lines.

### EXAMPLE 2

### Immunohistochemical analysis of the distribution of TFF3 in colorectal cancer

### I. MATERIAL AND METHODS

For the immunohistochemical study, 77 patients were chosen from the series of 129 with stages II/III of rectal cancer under 10 centimeters from the anal margin, who received two or more chemotherapy cycles and 50.4 Gy of radiotherapy before surgery, whose clinicopathological variables are recorded in the previous Table 1.

The same paraffined blocks which were used for the RNA extraction were used. 4 µm sections of these blocks were cut and adhered to silanized slides (Dako Corporation). These preparations were deparaffinized and the endogenous peroxidase was blocked by incubating in 3% H₂O₂ in methanol for 10 minutes at room temperature. The antigenic demasking was carried out by means of incubation with EDTA for 45 minutes at 155°C. The commercial monoclonal antibody against TFF3 (Abnova H00007033-M01) was used at a 1:500 dilution in 1% BSA in TBS. The incubation was carried out for 30 minutes at room temperature. For the viewing of the reaction the EnVision^{™} kit system (Dako Corporation, Carpinteria, CA) which uses a secondary antibody bound to peroxidase was used. This secondary antibody was incubated for 30 minutes at room temperature. Finally, the preparations were incubated with the chromogenic substrate diaminobenzidine (Dako Corporation, Carpinteria, CA) for 5 minutes at room temperature. The preparations were counterstained with hematoxylin and the tissue was dehydrated using alcohols of increasing strength and xylol. The preparations were analyzed by a pathologist.

### II. RESULTS

### Immunohistochemistry of TFF3

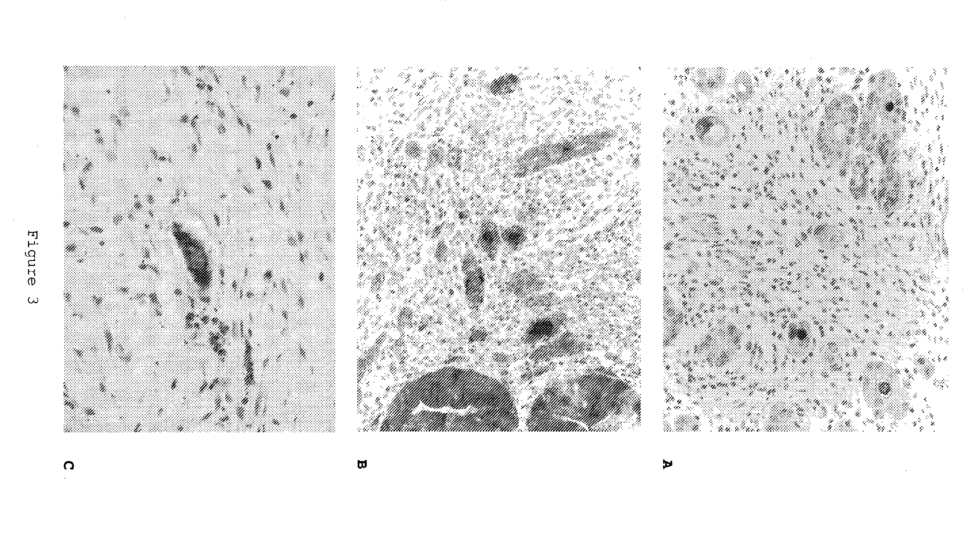

The immunohistochemical study was performed in samples from 77 patients. The rest of the patients of the series could not be studied due to the lack of sample. The complete sample (before and after the treatment) was analyzed in 18 patients. The preliminary analysis from these 18 patients did not show significant differences of expression of TFF3 upon comparing the samples before and after the treatment. Therefore, in the remaining 59 patients only the sample of the surgical specimen after CRT was studied. From the total of 77 surgical samples already treated, 65 had assessable immunostaining and showed, in some cases, the presence of intense immunostaining of TFF3 in the secretion in the lumen of the tumor microglands. The analysis performed of these immunostainings is limited to the assessment of whether or not there is presence of intense immunostaining of the secretion in the lumen of the microglands (Figure 3A and 3B). These 65 samples presented variable expression of TFF3 and from them the characteristic image of intense immunostaining of the secretion in microglands (Figure 3A and 3B) was found in 19 of the cases. The remaining 46 did not present immunostaining of the extracellular secretion. Out of 19 cases with secretion, 16 belong to patients who suffered from relapse of his/her disease. However only 10 of the remaining 46 relapsed. These results show a statistically significant relationship (P<0.0001) between the presence of this type of intense immunostaining of the secretion in microglands and the risk of relapse in the patients studied; this result is shown in Figure 2.

The patients who show intense immunostaining of the secretion showed a significant worse tumor response (Table 1), lower overall survival (Figure 3A) and lower disease-free survival (Figure 3B).

Furthermore, in some of the 19 cases with intense immunostaining of the secretion in microglands, they also showed immunostaining of TFF3 in the lumen of lymph and blood vessels (Figure 3C).

### Comparison of the result of the analysis of expression of TFF3 by means of CRT-PCR and immunohistochemistry

Of the 65 samples with expression assessable by means of immunohistochemistry of TFF3, 57 have the expression of TFF3 measured by means of quantitative PCR. These 57 patients were taken to analyze the possible correlation between the results of the measurement of expression of TFF3 with both techniques. The results show a significant correlation between the patients who increase the expression of TFF3 upon comparing the measurement before and after the treatment by means of quantitative PCR and the patients who showed intense immunostaining of the secretion in microglands.

### III. DISCUSSION

TFF3 has also been studied by means of immunohistochemistry and the results obtained by means of both techniques have been compared. The results of the analysis by means of immunohistochemical show, as the most significant finding upon studying the sample after the treatment, that some cases present positive immunostaining of TFF3 in the secretion to the lumen of the tumor microglands. The image of this immunostaining, which is shown in Figure 3, is very characteristic and allows obtaining an objectifiable analysis parameter. The onset of these secretions relates to a higher risk of relapse of the disease. Furthermore, the results show a statistically significant correlation upon comparing the expression obtained by means of analysis of the messenger RNA using quantitative PCR and the results obtained by analysis by means of immunohistochemistry of the protein. This result is very interesting, since this technique is usual for diagnosis in the Anatomic Pathology services of hospitals all over the world. Therefore, the use of this technique to analyze the presence or not of immunostaining of TFF3 in the extracellular secretions to the lumen of the tumor microglands in the surgical specimen after the treatment could have prognostic value in patients diagnosed with colorectal cancer.

## Claims

1. An *in vitro* method for establishing a prognosis concerning the progress of a patient diagnosed with colorectal cancer after the administration of a therapy, comprising
(i) quantifying the expression levels of the *TFF3* gene or
(ii) quantifying the expression levels of the protein encoded by said gene, or any amino acid sequence that has at least 90% identity with said TFF3 protein,
in a biological sample from said patient before and after the therapy, wherein an increase of the expression of *TFF3* or of the expression level of the protein encoded by said gene, or of any amino acid sequence that has at least 90% identity with said TFF3 protein, after the administered therapy with respect to the expression of *TFF3* or the expression level of the protein encoded by said gene, or any amino acid sequence that has at least 90% identity with said TFF3 protein, before the therapy, is indicative of a worse progress of the patient.

2. An *in vitro* method for monitoring the effect of a therapy administered to a patient diagnosed with colorectal cancer, comprising
(i) quantifying the expression levels of the *TFF3* gene or
(ii) quantifying the levels of the protein encoded by said gene, or any amino acid sequence that has at least 90% identity with-said TFF3 protein,
in a biological sample from said patient before and after said therapy, wherein an increase of the expression of *TFF3* or of the expression level of the protein encoded by said gene, or of any amino acid sequence that has at least 90% identity with said TFF3 protein, after the administered therapy with respect to the expression of *TFF3* or the expression level of the protein encoded by said gene, or any amino acid sequence that has at least 90% identity with-of said TFF3 protein, before the therapy, is indicative that the administered therapy is not effective.

3. An *in vitro* method for designing a customized therapy for a patient diagnosed with colorectal cancer, comprising
(i) quantifying the expression levels of the *TFF3* gene or
(ii) quantifying the levels of the protein encoded by said gene, or any amino acid sequence that has at least 90% identity with said TFF3 protein,
in a biological sample from said patient before and after the therapy, wherein an increase of the expression of *TFF3* or of the expression level of the protein encoded by said gene, or of any amino acid sequence that has at least 90% identity with said TFF3 protein, after the administered therapy with respect to the expression of *TFF3* or the expression level of the protein encoded by said gene, or any amino acid sequence that has at least 90% identity with said TFF3 protein, before the therapy, is indicative that the patient needs an alternative therapy to the therapy originally administered.

4. The method according to any of claims 1 to 3, wherein quantifying the expression levels of the *TFF3* gene comprises quantifying the messenger RNA (mRNA) of the *TFF3* gene, a fragment of said mRNA, complementary DNA (cDNA) of the *TFF3* gene, a fragment of said cDNA, or mixtures thereof or wherein quantifying the levels of the protein encoded by the *TFF3* gene comprises quantifying the TFF3 protein.

5. The method according to any of claims 1 to 4, wherein the administered therapy is a cytotoxic and/or cytostatic treatment, preferably chemotherapy, radiotherapy, antitumor agents or combinations thereof.

6. The method according to claim 5, wherein the chemotherapy comprises a compound selected from an alkylating agent preventing replication, a fluoropyrimidine, a thymidylate synthase inhibitor, a topoisomerase inhibitor and combinations thereof.

7. The method according to claim 6, wherein the alkylating agent preventing replication is oxaliplatin, the fluoropyrimidine is 5-fluorouracil, UFT, Utefos or capecitabine, the thymidylate synthase inhibitor is raltitrexed and the topoisomerase I inhibitor is irinotecan.

8. The method according to claim 5, wherein the antitumor agents comprise antiangiogenic agents and signaling pathway inhibitors.

9. The method according to claim 8, wherein the antiangiogenic agent is bevacizumab and the signaling pathway inhibitor is cetuximab, panitumumab, or erlotinib.

10. The method according to any of claims 1 to 8, wherein the biological sample is a tissue sample or a biological fluid.

11. The method according to claim 10, wherein the tissue sample is tumor tissue sample.

12. An *in vitro* method for establishing a prognosis concerning the progress of a patient diagnosed with colorectal cancer, comprising determining the distribution pattern of the protein encoded by the *TFF3* gene, or any amino acid sequence that has at least 90% identity with said TFF3 protein, in the tumor, wherein the detection of TFF3, or any amino acid sequence that has at least 90% identity with said TFF3 protein, in the lumen of the tumor microglands and/or inside the lymph or blood vessels is indicative of a worse progress of a patient.

13. The method according to claim 1 or 12, wherein the progress of a patient diagnosed with colorectal cancer is determined using a parameter selected from the group of risk of relapse, disease-free survival and/or overall survival of the patient.

14. The method according to claim 12 or 13, wherein chemoradiotherapy has been administered to the patient diagnosed with colorectal cancer before the extraction of the tumor.

15. The method according to claim 12 or 14, wherein the detection of the protein encoded by the *TFF3* gene is performed by means of immunohistochemistry.

16. The method according to any of claims 1 to 11 or 12 to 15, wherein the colorectal cancer is large intestine adenocarcinoma in stages II or III or IV.

## Patentansprüche

1. *In vitro* Verfahren zum Erstellen einer Prognose über den Fortschritt eines mit Darmkrebs diagnostizierten Patienten nach dem Verabreichen einer Therapie, umfassend
(i) Quantifizieren der Expressionslevel des *TFF3* Gens oder
(ii) Quantifizieren der Expressionslevel des durch das Gen kodierten Proteins oder einer Aminosäuresequenz, die mindestens 90% Identität mit dem TFF3 Protein hat,
in einer biologischen Probe des Patienten vor und nach der Therapie, wobei eine Zunahme der Expression von *TFF3* oder des Expressionslevels des durch das Gen kodierten Proteins oder einer Aminosäuresequenz, die mindestens 90% Identität mit dem TFF3 Protein hat, nach dem Verabreichen der Therapie gegenüber der Expression des *TFF3* oder dem Expressionslevel des durch das Gen kodierten Proteins oder einer Aminosäuresequenz, die mindestens 90% Identität mit dem TFF3 Protein hat, vor der Therapie, einen schlechteren Fortschritt des Patienten anzeigt.

2. *In vitro* Verfahren zur Überwachung der Wirkung einer Therapie, die einem mit Darmkrebs diagnostizierten Patienten verabreicht wird, umfassend
(i) Quantifizieren der Expressionslevel des *TFF3* Gens oder
(ii) Quantifizieren der Level des durch das Gen kodierten Proteins oder einer Aminosäuresequenz, die mindestens 90% Identität mit dem TFF3 Protein hat,
in einer biologischen Probe des Patienten vor und nach der Therapie, wobei eine Zunahme der Expression von *TFF3* oder des Expressionslevels des durch das Gen kodierten Proteins oder einer Aminosäuresequenz, die mindestens 90% Identität mit dem TFF3 Protein hat, nach dem Verabreichen der Therapie gegenüber der Expression von *TFF3* oder dem Expressionslevel des durch das Gen kodierten Proteins oder einer Aminosäuresequenz, die mindestens 90% Identität mit dem TFF3 Protein hat, vor der Therapie, anzeigt, dass die verabreichte Therapie nicht wirksam ist.

3. *In vitro* Verfahren zum Entwickeln einer individuellen Therapie für einen mit Darmkrebs diagnostizierten Patienten, umfassend
(i) Quantifizieren der Expressionslevel des *TFF3* Gens oder
(ii) Quantifizieren der Level des durch das Gen kodierten Proteins oder einer Aminosäuresequenz, die mindestens 90% Identität mit dem TFF3 Protein hat,
in einer biologischen Probe des Patienten vor und nach der Therapie, wobei eine Zunahme der Expression von *TFF3* oder des Expressionslevels des durch das Gen kodierten Proteins oder einer Aminosäuresequenz, die mindestens 90% Identität mit dem TFF3 Protein hat, nach dem Verabreichen der Therapie gegenüber der Expression von *TFF3* oder dem Expressionslevel des durch das Gen kodierten Proteins oder eine Aminosäuresequenz, die mindestens 90% Identität mit dem TFF3 Protein hat, vor der Therapie, anzeigt, dass der Patient eine alternative Therapie zu der ursprünglich verabreichten Therapie benötigt.

4. Verfahren nach einem der Ansprüche 1 bis 3, wobei das Quantifizieren der Expressionslevel des *TFF3* Gens das Quantifizieren der Boten-RNA (mRNA) des *TFF3* Gens, eines Fragmentes der mRNA, komplementärer DNA (cDNA) des *TFF3* Gens, eines Fragmentes der cDNA oder Mischungen davon umfasst oder wobei das Quantifizieren der Level des durch das *TFF3* Gen kodierten Proteins das Quantifizieren des TFF3 Proteins umfasst.

5. Verfahren nach einem der Ansprüche 1 bis 4, wobei die verabreichte Therapie eine zytotoxische und/oder zytostatische Behandlung ist, vorzugsweise Chemotherapie, Radiotherapie, Antitumor-Mittel oder Kombinationen davon.

6. Verfahren nach Anspruch 5, wobei die Chemotherapie eine Verbindung umfasst, die aus einem Alkylierungsmittel, das Replikation verhindert, einem Fluoropyrimidin, einem Thymidylat-Synthase-Inhibitor, einem Topoisomerase-Inhibitor und Kombinationen davon ausgewählt wird.

7. Verfahren nach Anspruch 6, wobei das Alkylierungsmittel, das Replikation verhindert, Oxaliplatin ist, das Fluoropyrimidin 5-Fluorouracil, UFT, Utefos oder Capecitabin ist, der Thymidylat-Synthase-Inhibitor Raltitrexed ist und der Topoisomerase I Inhibitor Irinotecan ist.

8. Verfahren nach Anspruch 5, wobei die Antitumor-Mittel, antiangiogene Mittel und Signalweg-Inhibitoren umfassen.

9. Verfahren nach Anspruch 8, wobei das antiangiogene Mittel Bevacizumab ist und der Signalweg-Inhibitor Cetuximab, Panitumumab oder Erlotinib ist.

10. Verfahren nach einem der Ansprüche 1 bis 8, wobei die biologische Probe eine Gewebeprobe oder eine biologische Flüssigkeit ist.

11. Verfahren nach Anspruch 10, wobei die Gewebeprobe eine Tumorgewebeprobe ist.

12. *In vitro* Verfahren zum Erstellen einer Prognose über den Fortschritt eines mit Darmkrebs diagnostizierten Patienten, umfassend das Bestimmen des Verteilungsmusters des durch das *TFF3* Gen kodierten Proteins, oder einer Aminosäuresequenz, die mindestens 90% Identität mit dem TFF3 Protein hat, in dem Tumor, wobei die Detektion des TFF3 oder einer Aminosäuresequenz, die mindestens 90% Identität mit dem TFF3 Protein hat, im Lumen der Tumormikrodrüsen und/oder innerhalb der Lymphe oder Blutgefäße einen schlechteren Fortschritt des Patienten anzeigt.

13. Verfahren nach Anspruch 1 oder 12, wobei der Fortschritt eines mit Darmkrebs diagnostizierten Patienten mit einem aus der Gruppe aus Rückfallrisiko, krankheitsfreiem Überleben und/oder Gesamtüberleben des Patienten ausgewählten Parameters bestimmt wird.

14. Verfahren nach Anspruch 12 oder 13, wobei dem mit Darmkrebs diagnostizierten Patienten vor der Extraktion des Tumors eine Chemo-Strahlentherapie verabreicht wurde.

15. Verfahren nach Anspruch 12 oder 14, wobei die Bestimmung des durch das *TFF3* Gen kodierten Proteins mittels Immunhistochemie erfolgt.

16. Verfahren nach einem der Ansprüche 1 bis 11 oder 12 bis 15, wobei der Darmkrebs ein Dickdarm-Adenokarzinom im Stadium II oder III oder IV ist.

## Revendications

1. Procédé *in vitro* permettant d'établir un pronostic concernant la progression d'un patient auquel a été diagnostiqué un cancer colorectal après l'administration d'une thérapie, comprenant
(i) la quantification des niveaux d'expression du gène *TFF3* ou
(ii) la quantification des niveaux d'expression de la protéine codée par ledit gène, ou de toute séquence d'acides aminés qui a au moins 90 % d'identité avec ladite protéine TFF3,
dans un échantillon biologique provenant dudit patient avant et après la thérapie, où une augmentation de l'expression de *TFF3* ou du niveau d'expression de la protéine codée par ledit gène, ou de toute séquence d'acides aminés qui a au moins 90 % d'identité avec ladite protéine TFF3, après la thérapie administrée en ce qui concerne l'expression de *TFF3* ou le niveau d'expression de la protéine codée par ledit gène, ou de toute séquence d'acides aminés qui a au moins 90 % d'identité avec ladite protéine TFF3, avant la thérapie, indique une aggravation de l'état du patient.

2. Procédé *in vitro* permettant de surveiller l'effet d'une thérapie administrée à un patient auquel a été diagnostiqué un cancer colorectal, comprenant
(i) la quantification des niveaux d'expression du gène *TFF3* ou
(ii) la quantification des niveaux de la protéine codée par ledit gène, ou de toute séquence d'acides aminés qui a au moins 90 % d'identité avec ladite protéine TFF3,
dans un échantillon biologique provenant dudit patient avant et après ladite thérapie, où une augmentation de l'expression de *TFF3* ou du niveau d'expression de la protéine codée par ledit gène, ou de toute séquence d'acides aminés qui a au moins 90 % d'identité avec ladite protéine TFF3, après la thérapie administrée en ce qui concerne l'expression de *TFF3* ou le niveau d'expression de la protéine codée par ledit gène, ou de toute séquence d'acides aminés qui a au moins 90 % d'identité avec ladite protéine TFF3, avant la thérapie, indique que la thérapie administrée n'est pas efficace.

3. Procédé *in vitro* permettant de concevoir une thérapie personnalisée pour un patient auquel a été diagnostiqué un cancer colorectal, comprenant
(i) la quantification des niveaux d'expression du gène *TFF3* ou
(ii) la quantification des niveaux de la protéine codée par ledit gène, ou de toute séquence d'acides aminés qui a au moins 90 % d'identité avec ladite protéine TFF3,
dans un échantillon biologique provenant dudit patient avant et après la thérapie, où une augmentation de l'expression de *TFF3* ou du niveau d'expression de la protéine codée par ledit gène, ou de toute séquence d'acides aminés qui a au moins 90 % d'identité avec ladite protéine TFF3, après la thérapie administrée en ce qui concerne l'expression de *TFF3* ou le niveau d'expression de la protéine codée par ledit gène, ou de toute séquence d'acides aminés qui a au moins 90 % d'identité avec ladite protéine TFF3, avant la thérapie, indique que le patient a besoin d'une thérapie alternative à la thérapie administrée à l'origine.

4. Procédé selon l'une quelconque des revendications 1 à 3, dans lequel la quantification des niveaux d'expression du gène *TFF3* comprend la quantification de l'ARN messager (ARNm) du gène *TFF3,* d'un fragment dudit ARNm, de l'ADN complémentaire (ADNc) du gène *TFF3,* d'un fragment dudit ADNc, ou de mélanges de ceux-ci ou dans lequel la quantification des niveaux de la protéine codée par le gène *TFF3* comprend la quantification de la protéine TFF3.

5. Procédé selon l'une quelconque des revendications 1 à 4, dans lequel la thérapie administrée est un traitement cytotoxique et/ou cytostatique, de préférence une chimiothérapie, une radiothérapie, des agents antitumoraux ou des combinaisons de ceux-ci.

6. Procédé selon la revendication 5, dans lequel la chimiothérapie comprend un composé sélectionné parmi un agent alkylant empêchant la réplication, une fluoropyrimidine, un inhibiteur de la thymidylate synthase, un inhibiteur de la topoisomérase et des combinaisons de ceux-ci.

7. Procédé selon la revendication 6, dans lequel l'agent alkylant empêchant la réplication est l'oxaliplatine, la fluoropyrimidine est le 5-fluorouracile, UFT, Utefos ou la capécitabine, l'inhibiteur de la thymidylate synthase inhibitor est le raltitrexed et l'inhibiteur de la topoisomerase I est l'irinotécan.

8. Procédé selon la revendication 5, dans lequel les agents antitumoraux comprennent des agents antiangiogenèse et des inhibiteurs des voies de signalisation.

9. Procédé selon la revendication 8, dans lequel l'agent antiangiogenèse est le bevacizumab et l'inhibiteur des voies de signalisation est le cetuximab, la panitumumab ou l'erlotinib.

10. Procédé selon l'une quelconque des revendications 1 à 8, dans lequel l'échantillon biologique est un échantillon de tissu ou un fluide biologique.

11. Procédé selon la revendication 10, dans lequel l'échantillon de tissu est un échantillon de tissu tumoral.

12. Procédé *in vitro* permettant d'établir un pronostic concernant la progression d'un patient auquel a été diagnostiqué un cancer colorectal, comprenant la détermination du profil de distribution de la protéine codée par le gène *TFF3,* ou de toute séquence d'acides aminés qui a au moins 90 % d'identité avec ladite protéine TFF3, dans la tumeur, dans lequel la détection de TFF3, ou de toute séquence d'acides aminés qui a au moins 90 % d'identité avec ladite protéine TFF3, dans la lumière des microglandes tumorales et/ou à l'intérieur des vaisseaux lymphatiques ou sanguins indique une aggravation de l'état d'un patient.

13. Procédé selon la revendication 1 ou 12, dans lequel la progression d'un patient auquel a été diagnostiqué un cancer colorectal est déterminée à l'aide d'un paramètre sélectionné dans le groupe constitué du risque de récidive, de la survie sans récidive et/ou la survie globale du patient.

14. Procédé selon la revendication 12 ou 13, dans lequel la chimiothérapie a été administrée au patient auquel a été diagnostiqué un cancer colorectal avant l'ablation de la tumeur.

15. Procédé selon la revendication 12 ou 14, dans lequel la détection de la protéine codée par le gène *TFF3* est réalisée au moyen d'une immunohistochimie.

16. Procédé selon l'une quelconque des revendications 1 à 11 ou 12 à 15, dans lequel le cancer colorectal est un adénocarcinome du gros intestin aux stades II ou III ou IV.
